# EUROPEAN PATENT APPLICATION

(11) **EP 4 170 020 A1**
(43) Date of publication of application: **26.04.2023**
(21) Application number: 21826003.2
(22) Date of filing: 16.06.2021
(51) Int. Cl.: C12N 5/10, A61K 35/17, A61P 35/00, C12N 5/0783, C12N 15/12, C12N 15/19, C12N 15/09

(54) **CHIMERIC ANTIGEN RECEPTOR-EXPRESSING IMMUNOCOMPETENT CELLS**

(30) Priority: 17.06.2020 JP 2020104365
(71) Applicant: Kyoto University, Kyoto-shi, Kyoto 606-8501 (JP)
(72) Inventor: KANEKO, Shin, Kyoto-shi, Kyoto 606-8501 (JP); UEDA, Tatsuki, Kyoto-shi, Kyoto 606-8501 (JP)
(74) Representative: Hoffmann Eitle
(86) International application number: PCT/JP2021/022944
(87) International publication number: WO 2021/256522

(57) **Abstract**

The present invention aims to increase cytotoxic activity of an immunocompetent cell to thereby enhance a therapeutic effect against diseases such as cancers, and provides an immunocompetent cell having decreased diacylglycerol kinase activity, which cell expresses a fusion protein including IL-15 and an IL-15 receptor α subunit, and which cell expresses a chimeric antigen receptor.

## Description

### TECHNICAL FIELD

The present invention relates to an immunocompetent cell that expresses a chimeric antigen receptor, and a pharmaceutical comprising it.

### BACKGROUND ART

In recent years, immune cell therapy has been attracting attention as a therapeutic method for cancer. Immune cell therapy is a therapeutic method in which immune cells grown and activated outside the body of a patient are administered to the patient to allow the immune cells to attack cancer cells. Unlike the conventional three major therapeutic methods, that is, surgical treatment, radiation therapy, and chemotherapy, immune cell therapy has an advantage in that it hardly shows side effects. Immune cell therapy includes a variety of types of therapeutic methods. Among these, treatment using T cells (cytotoxic T cells: CTLs), which are responsible for natural immunity and have cytotoxic activity against cancer cells, is attracting attention.

Further, in order to allow cytotoxic T cells to recognize and attack a specific antigen expressed on the surface of cancer cells, expression of a chimeric antigen receptor (CAR) on the surface of the cytotoxic T cells has also been carried out.

Patent Documents 1 and 2 disclose methods of inducing CD8-positive cytotoxic T cells from pluripotent stem cells through hematopoietic progenitor cells and CD4/CD8 double positive T cells, and Patent Document 1 discloses that a chimeric antigen receptor may be expressed on the CD8-positive cytotoxic T cells.

Patent Document 3 discloses a method of inducing cytotoxic T cells from pluripotent stem cells, and also discloses that a chimeric antigen receptor may be expressed on the cytotoxic T cells, and that a fusion protein containing interleukin 15 (IL-15) and an interleukin 15 receptor α subunit (IL-15Rα) may be expressed on the cytotoxic T cells. The fusion protein is also disclosed in Non-patent Document 1.

On the other hand, Non-patent Document 2 describes that, by knockout of the diacylglycerol kinase gene in cytotoxic T cells, anticancer activity of the cytotoxic T cells can be enhanced.

However, there has been no case where expression of a fusion protein of IL-15 and IL-15Rα is combined with knockout of the diacylglycerol kinase gene in immunocompetent cells such as T cells. There has been no motivation to combine these among the variety of known modifications of immunocompetent cells, and no effect has been known for the combination.

### PRIOR ART DOCUMENTS

### [Patent Documents]

[Patent Document 1] WO 2017/221975
[Patent Document 2] WO 2018/135646
[Patent Document 3] WO 2020/013315

### [Non-patent Documents]

[Non-patent Document 1] Proc. Natl. Acad. Sci. 113, E7788-e7797 (2016)
[Non-patent Document 2] Cancer Res. 2018 Aug 15; 78(16):4692-4703.

### SUMMARY OF THE INVENTION

### PROBLEMS TO BE SOLVED BY THE INVENTION

An object of the present invention is to increase cytotoxic activity of an immunocompetent cell to thereby enhance a therapeutic effect against diseases such as cancer.

### MEANS FOR SOLVING THE PROBLEMS

The present inventors intensively studied to solve the above problem. As a result, the present inventors discovered that, by decreasing the diacylglycerol kinase activity and allowing expression of a fusion protein (IL-15/IL-15Rα) containing IL-15 and IL-15Rα in immunocompetent cells such as cytotoxic T cells that express a chimeric antigen receptor, cytotoxicity against cancer cells and the like can be remarkably improved to increase the therapeutic effect, thereby completing the present invention.

The following is an outline of the present invention.
[1] An immunocompetent cell having decreased diacylglycerol kinase activity, wherein said cell expresses a fusion protein (IL-15/IL-15Rα) comprising IL-15 and an IL-15 receptor α subunit (IL-15Rα), and expresses a chimeric antigen receptor.
[2] The immunocompetent cell according to [1], wherein said cell comprises one or more kinds of diacylglycerol kinase genes having a mutation(s) that decrease(s) diacylglycerol kinase activity in a cell.
[3] The immunocompetent cell according to [1] or [2], wherein the fusion protein is a transmembrane protein.
[4] The immunocompetent cell according to any one of [1] to [3], wherein said cell is CD5-positive and CD8β-positive.
[5] The immunocompetent cell according to any one of [1] to [4], wherein said cell is a T cell.
[6] The immunocompetent cell according to any one of [1] to [5], wherein said cell is obtainable by differentiation induction from a pluripotent stem cell.
[7] The immunocompetent cell according to [6], wherein the pluripotent stem cell is an induced pluripotent stem cell.
[8] A pharmaceutical composition comprising the immunocompetent cell according to any one of [1] to [7].
[9] The pharmaceutical composition according to [8], for treatment of cancer.
[10] The pharmaceutical composition according to [9], wherein the cancer is a solid cancer.
[11] A method for treating cancer, comprising the step of administering a therapeutically effective amount of the immunocompetent cell according to any one of [1] to [7] to a subject in need of treatment.
[12] Use of the immunocompetent cell according to any one of [1] to [7] in the manufacture of a pharmaceutical composition for cancer treatment.
[13] The immunocompetent cell according to any one of [1] to [7] for use intreatment of cancer.
[14] A method of producing an immunocompetent cell that expresses a chimeric antigen receptor, comprising the step of:
   (a) providing an immunocompetent cell that expresses a chimeric antigen receptor;
   wherein the immunocompetent cell has decreased diacylglycerol kinase activity, and expresses a fusion protein (IL-15/IL-15Rα) comprising IL-15 and an IL-15 receptor α subunit (IL-15Rα).
[15] The method according to [14], comprising the step of:
   (b) allowing a pluripotent stem cell to differentiate into an immunocompetent cell;
   before Step (a).
[16] The method according to [15], wherein the cell obtained in Step (b) expresses a chimeric antigen receptor.
[17] The method according to [16], wherein a pluripotent stem cell that expresses a chimeric antigen receptor is used in Step (b), or a chimeric antigen receptor is allowed to be expressed in any stage of Step (b).
[18] The method according to any one of [15] to [17], wherein the cell obtained in Step (b) has decreased diacylglycerol kinase activity.
[19] The method according to [18], wherein a pluripotent stem cell having decreased diacylglycerol kinase activity is used in Step (b), or a modification that decreases diacylglycerol kinase activity is carried out in any stage of Step (b).
[20] The method according to any one of [15] to [19], wherein the cell obtained in Step (b) expresses a fusion protein (IL-15/IL-15Rα) comprising IL-15 and an IL-15 receptor α subunit (IL-15Rα).
[21] The method according to [20], wherein a pluripotent stem cell that expresses IL-15/IL-15Rα is used in Step (b), or IL-15/IL-15Rα is allowed to be expressed in any stage of Step (b).

### EFFECT OF THE INVENTION

By the present invention, a remarkably excellent cytotoxic effect can be exerted in immune-cell therapy for cancer or the like. Further, by preparing immunocompetent cells such as cytotoxic T cells from pluripotent stem cells, cells required for the treatment can be stably supplied.

### BRIEF DESCRIPTION OF THE DRAWINGS

Fig. 1 is a schematic diagram of a CAR introduction vector used in Examples.
Fig. 2Ais a diagram illustrating the result of detection of phosphorylated ERK (pERK) in iCAR-T_{CTL}, DGK-dKO iCAR-T_{CTL}, and pCAR-T_{CTL} after 10 minutes of co-culture with irradiated SK-Hep-GPC3.
Fig. 2B is a diagram illustrating gRNA (sgRNA) sequences for the DGKα (DGKa) or DGKζ (DGKz) gene, and the sequences of the DGKα and DGKζ genes whose both alleles are mutated in DGK-dKO iCAR. Each bar indicates a mutated sequence.
Fig. 2C is a graph illustrating the growth of iCAR-T_{CTL} and DGK-dKO iCAR-T_{CTL} through target cells. The cells were co-cultured with irradiated SK-Hep-GPC3, and measurement was carried out at Hour 72 using a standard ³H-thymidine incorporation assay (n=3, mean ± SEM).
Fig. 2D is a photographic diagram illustrating the result of in vivo imaging of iCAR-T_{CTL} or DGK-dKO iCAR-T_{CTL} injected to JHH7 heterologous transplantation mice (n=8/group) based on luciferase at each indicated time point.
Fig. 2E is a graph illustrating changes in the expression level of luciferase over time in iCAR-T_{CTL} or DGK-dKO iCAR-T_{CTL} injected to JHH7 heterologous transplantation mice (n=8/group), wherein the expression level was measured at each indicated time point.
Fig. 2F is a photographic diagram illustrating the result of in vivo bioluminescence imaging of luciferase-labeled KOC7c in NSG mice treated with PBS, iCAR-T_{CTL}, DGK-dKO iCAR-T_{CTL}, or pCAR-T_{CTL} (n=8/group).
Fig. 2G is a graph illustrating the total body flux per individual as the total tumor volume of luciferase-labeled KOC7c in NSG mice treated with PBS, iCAR-T_{CTL}, DGK-dKO iCAR-T_{CTL}, or pCAR-T_{CTL} (n=8/group).
Fig. 2H is a graph illustrating the result of evaluation of changes in the survival rate in NSG mice treated with PBS, iCAR-T_{CTL}, DGK-dKO iCAR-T_{CTL}, or pCAR-T_{CTL} (n=8/group), wherein the survival rate was measured at each indicated time point after the injection (***p<0.01, ****p<0.001).
Fig. 3A is a diagram illustrating a treatment schedule for a KOC7c ovarian cancer cell peritoneal transplantation model.
Fig. 3B is a photographic diagram illustrating the result of in vivo bioluminescence imaging of a heterologously transplanted tumor KOC7c labeled with renilla luciferase in NSG mice. After intraperitoneal injection of 5×10⁵ luciferase-expressing KOC7c cells to each NSG mouse, 1×10⁶ cells of iCAR-T_{CTL}, DGK-dKO iCAR-T_{CTL}, pCAR-T_{CTL}, or DGK-pKO pCAR-T_{CTL}(all of these were genetically modified with mbIL-15) were intraperitoneally injected once (n=5/group) from Day 3.
Fig. 3C is a graph illustrating the result of evaluation of changes in the survival rate of KOC7c transplantation mice treated by administration of each type of T_{CTL}, wherein the survival rate was measured at each indicated time point after the injection (***p<0.001, ^{∗∗∗∗}p<0.0001).
Fig. 3D is a photographic diagram illustrating in vivo bioluminescence imaging of the injected iCAR-T_{CTL} mbIL15, DGK-dKO iCAR-T_{CTL} mbIL15, pCAR-T_{CTL} mbIL15, and DGK-pKO pCAR-T_{CTL} mbIL 15 (all of these were labeled with renilla luciferase) on Day 13 and Day 20.
Fig. 3E is a photographic diagram illustrating the result of quantification on Day 13 and Day 20 in in vivo bioluminescence imaging of the injected iCAR-T_{CTL} mbIL15, DGK-dKO iCAR-T_{CTL} mbIL15, pCAR-T_{CTL} mbIL15, and DGK-pKO pCAR-T_{CTL} mbIL 15 (all of these were labeled with renilla luciferase) (**p<0.01, ^{∗∗∗}p<0.001).
Fig. 3F is a diagram illustrating a treatment schedule for a liver cancer heterologous transplantation model.
Fig. 3G is a graph illustrating changes in the subcutaneous tumor volume in each individual after the liver cancer heterologous transplantation model was treated with each type of T_{CTL} cells. After subcutaneous inoculation of 2×10⁵ JHH7 cells to each NSG mouse, 1×10⁶ cells of iCAR-T_{CTL} mbIL15, DGK-dKO iCAR-T_{CTL} mbIL15, pCAR-T_{CTL} mbIL15, or DGK-pKO pCAR-T_{CTL} mbIL15 were intravenously administered (n=5/group) on Day 3.
Fig. 3H is a graph illustrating changes in the subcutaneous tumor volume in each group after the liver cancer heterologous transplantation model was treated with each type of T_{CTL} cells (mean ± SEM).
Fig. 3I is a graph illustrating the result of evaluation of changes in the survival rate after the liver cancer heterologous transplantation model was treated with each type of T_{CTL} cells (**p<0.01).
Fig. 3J is a diagram illustrating the result of analysis of peripheral blood of the liver cancer heterologous transplantation model by FCM (flow cytometry) on Day 28 after the injection of the T cells.
Fig. 4Ais a diagram illustrating a treatment schedule for a liver cancer heterologous transplantation model.
Fig. 4B is a graph illustrating changes in the subcutaneous tumor volume in each individual after the liver cancer heterologous transplantation model was treated with each type of T_{CTL} cells. After subcutaneous inoculation of 5×10⁵ SK-Hep-GPC3 cells to each NSG mouse, 1×10⁷ cells of pCAR-T_{CTL}, DGK-pKO pCAR-T_{CTL} mbIL15, or DGK-dKO iCAR-T_{CTL} mbIL15 were intravenously administered (n=6/group) on Day 10.
Fig. 4C is a graph illustrating changes in the subcutaneous tumor volume in each group after the liver cancer heterologous transplantation model was treated with each type of T_{CTL} cells (mean ± SEM; **p<0.01, ****p<0.0001).
Fig. 5A is a diagram illustrating a treatment schedule for a KOC7c ovarian cancer cell peritoneal transplantation model.
Fig. 5B is a graph illustrating the result of evaluation of changes in the survival rate of KOC7c transplantation mice treated with each type of T_{CTL} cells (*p<0.05, **p<0.01).
Fig. 5C is a photographic diagram illustrating in vivo bioluminescence imaging of the injected DGKdKO iCAR-T_{CTL} mbIL15tg and DGKpKO pCAR-T_{CTL} mbIL15tg (both of these were labeled with renilla luciferase) on Days 7, 14, 21, 28, 42, and 74.
Fig. 5D is a diagram illustrating the result of analysis of effector memory phenotype-related factors by FCM that was carried out on Day 80 after the injection of the T cells, which analysis was carried out using ascites of KOC7c transplantation mice in the DGK-dKO iCAR-T_{CTL} mbIL15 group.

### MODE FOR CARRYING OUT THE INVENTION

The immunocompetent cell of the present invention has decreased diacylglycerol kinase activity. The cell expresses a fusion protein (IL-15/IL-15Rα) comprising IL-15 and IL-15Rα, and expresses a chimeric antigen receptor (CAR).

In the present description, "immunocompetent cell" means a cell responsible for immune function in a living body. Examples of the immunocompetent cell include lymphoid cells such as T cells, natural killer cells (NK cells), and B cells; antigen-presenting cells such as monocytes, macrophages, and dendritic cells; and granulocytes such as neutrophils, eosinophils, basophils, and mast cells. Preferred specific examples of the immunocompetent cell include T cells derived from mammals such as humans, dogs, cats, pigs, and mice, preferably T cells derived from humans. The T cells can be obtained by isolation and purification from a body fluid such as blood or bone marrow fluid; or from immunocompetent cells that infiltrate a tissue such as the spleen, thymus, or lymph node, or a cancer tissue such as a primary tumor, metastatic tumor, or cancerous ascites. T cells prepared from ES cells or iPS cells (iPSCs) may also be used. Examples of such T cells include αβ T cells, γδ T cells, CD8⁺ T cells, CD4⁺ T cells (CD4⁺ helper T cells), tumor-infiltrating T cells, memory T cells, naive T cells, and NK T cells. The origin of the immunocompetent cell and the subject to whom the cell is administered may be either the same or different. In cases where the subject to whom the cell is administered is a human, the immunocompetent cell employed may be an autologous cell collected from the patient himself who is the target of the administration, or may be an allogeneic cell collected from another individual. Thus, the donor and the recipient may be either the same or different, but are preferably the same.

Preferred examples of the subject to whom the cell is administered include mammals and mammalian cells. More preferred examples of the mammals include humans, mice, dogs, rats, guinea pigs, rabbits, birds, sheep, pigs, cows, horses, cats, monkeys, and chimpanzees. Especially preferred examples of the mammals include humans.

The immunocompetent cell is not limited as long as it is an immunocompetent cell capable of expressing CAR. Since CAR is usually not naturally present, the immunocompetent cell is an immunocompetent cell capable of expressing exogenous CAR rather than endogenous CAR. The immunocompetent cell used in the present invention may express IL-7 and/or CCL19. In cases where the immunocompetent cell is a cell in which expression of IL-7 and/or CCL19 is no found, such as a T cell, or in cases where the immunocompetent cell is a cell which is not a T cell and whose expression of IL-7 and/or CCL19 is low, the immunocompetent cell used in the present invention may be a cell that expresses exogenous IL-7 and/or CCL19.

### <Cytotoxic T Cell>

The immunocompetent cell is especially preferably a cytotoxic T cell. A cytotoxic T cell (CTL) is a T cell that specifically exerts cytotoxic activity against a foreign cell such as a cancer cell presenting an antigen peptide. The cytotoxic activity can be confirmed using, for example, secretion or production of granzyme, perforin, or the like as an index.

The origin of the cytotoxic T cell is not limited. The cytotoxic T cell may be a cytotoxic T cell isolated from a living body, or may be a cytotoxic T cell obtained by differentiation induction from a pluripotent stem cell.

The cytotoxic T cell is preferably a CD8β-positive cell, more preferably a CD8β/CD5 double positive cell from the viewpoint of enhancing trafficking and cytotoxicity of the cytotoxic T cell. Further, the cytotoxic T cell may be CD3-positive and/or CD8α-positive. Further, the cell may be positive for α and β of the T-cell receptor.

The fact that the cells are positive for these molecules, that is, the fact that these molecules are expressed, can be confirmed by detection or labeling using antibodies against these molecules, sorting by flow cytometry, or the like.

### <Chimeric Antigen Receptor>

A chimeric antigen receptor (CAR) means a fusion protein comprising an antigen-binding domain, a transmembrane domain, and an intracellular signal transduction domain.

The antigen-binding domain of CAR may be a short-chain antibody (scFv) in which the light chain (VL) and heavy chain (VH) of the variable region of an antibody are linearly linked through a spacer such as a peptide linker (for example, a GS linker composed of glycine and serine). A T cell expressing CAR recognizes an antigen through the antigen-binding domain, and then the recognition signal is transduced into the T cell through the intracellular signal transduction domain. By introduction of the CAR to a T cell, specificity to a target antigen can be imparted. Further, since CAR is capable of directly recognizing an antigen molecule independently of HLA class I or class II, even a cell showing decreased expression of the HLA class I or class II gene can be allowed to cause high immune reaction.

Examples of the antigen include tumor antigens and pathogen antigens. Examples of the tumor antigens include one or more of glypican 3 (GPC3), CD 19, MUC16, MUC1, CAlX, CEA, CDS, CD7, CD10, CD20, CD22, CD30, CD33, CD34, CD38, CD41, CD44, CD49F, CD56, CD74, CD133, CD138, cytomegalovirus (CMV)-infected cell antigen, EGP-2, EGP-40, EpCAM, erb-B2,3,4, FBP, embryonic acetylcholine receptor, folate receptor-α, GD2, GD3, HER-2, hTERT, IL-13R-A2, κ-light chain, KDR, LeY, L1 cell adhesion molecule, MAGE-A1, mesothelin, NKG2D ligand, NY-ES0-1, carcinoembryonic antigen (h5T4), PSCA, PSMA, ROR1, TAG-72, VEGF-R2, and WT-1.

The transmembrane domain of CAR is not limited as long as it is a peptide capable of penetrating the cell membrane. Examples of the transmembrane domain include, but are not limited to, transmembrane domains derived from one or more kinds of proteins selected from the group consisting of α-chain, β-chain, or ζ chain of TCR, CD28, CD3 ε-chain, CD45, CD4, CD5, CD8, CD9, CD16, CD22, CD33, CD37, CD64, CD80, CD86, CD134, 4-1BB (CD137), CD154, ICOS, EGFR (epidermal growth factor receptor), and GITR. The transmembrane domain of the molecule from which the first intracellular signal transduction domain linked to the antigen-binding domain is derived may also be used. For example, in cases where the molecule from which the first intracellular signal transduction domain linked to the antigen-binding domain is derived is CD28, the transmembrane domain may also be derived from CD28. Alternatively, an artificially designed transmembrane domain may be used.

The intracellular signal transduction domain of the CAR is not limited as long as the region enables signal transduction into the immunocompetent cell when the antigen-binding domain is bound to the antigen. Examples of the intracellular signal transduction domain include, but are not limited to, intracellular domains derived from one or more kinds of proteins selected from the group consisting of the CD3ζ chain (TCRζ chain), FcRy chain, FcRβ chain, CD3γ chain, CD3δ chain, CD3ε chain, CD5, CD22, CD79a, CD79b, and CD66D. Among these, an intracellular signal transduction domain derived from the CD3ζ chain is preferred. The intracellular signal transduction domain may also contain an intracellular domain of a costimulatory molecule, and examples of the costimulatory molecule include intracellular domains derived from one or more kinds of proteins selected from the group consisting of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, and CD83. By selecting the type(s) and the number of the costimulatory molecule(s), strength and duration of the activity of the CAR can be controlled (for example, Mol Ther. 2009; 17:1453-1464).

A spacer may be incorporated between the antigen-binding domain and the transmembrane domain of the CAR, or between the intracellular signal transduction domain and the transmembrane domain of the CAR. As the spacer, a peptide composed of usually not more than 300 amino acids, preferably 10 to 100 amino acids, most preferably 25 to 50 amino acids may be used. Specific examples of the spacer include, but are not limited to, a hinge region derived from IgG1 or IgG4, and a peptide containing the CH2CH3 region of an immunoglobulin and part of CD3.

Specific examples of the CAR include, but are not limited to, a first-generation CAR in which scFV and a CD3ζ chain are linked to each other through a spacer, a second-generation CAR in which a transmembrane domain and an intracellular domain derived from CD28 are incorporated between the scFV and the CD3ζ chain of the first-generation CAR, a third-generation CAR in which an intracellular domain of a costimulatory molecule that is different from CD28 (4-1BB or OX40) is incorporated between the intracellular domain of CD28 and the CD3ζ chain in the second-generation CAR, and a fourth-generation CAR in which a cytokine or a molecule downstream of a cytokine receptor is linked to the second-generation CAR in order to obtain more sustained T cell activation.

More specific examples of the CAR include a chimeric antigen receptor comprising: as an antigen-binding domain, an scFv that recognizes GPC3 (for example, an scFv derived from the antibody GC33) or an scFv that recognizes CD19; as a transmembrane domain, a transmembrane domain of CD8; and, as intracellular signal transduction domains, an intracellular domain derived from CD28, an intracellular domain derived from CD30, an intracellular domain derived from 4-1BB, and/or an intracellular domain derived from the CD3ζ chain. The order of the above-described intracellular domains contained in the intracellular signal transduction domains is not limited. For example, they are contained in the order of the intracellular domain derived from CD28, the intracellular domain derived from CD30 or the intracellular domain derived from 4-1BB, and the intracellular domain derived from the CD3ζ chain.

As described later, the CAR can be expressed by allowing the immunocompetent cell to comprise a polynucleotide encoding the CAR.

### <Fusion Protein Comprising IL-15 and IL-15Rα>

The fusion protein (IL-15/IL-15Rα) comprising IL-15 and IL-15Rα may be either a transmembrane protein (which is sometimes called mbIL15) or a secretory protein. It is known that, in IL-15Rα, the IL-15-binding domain, which corresponds to the 1st to 65th amino acids from the N-terminus of the mature protein, is the responsible region for binding to IL-15 (Wei X. et al., J. Immunol., 167:277-282, 2001). Thus, the transmembrane protein may be a fusion protein of an IL-15Rα protein retaining the IL-15-binding domain and the transmembrane domain, and IL-15. On the other hand, the secretory protein may be a fusion protein of an IL-15Rα protein which retains the IL-15-binding domain, but which lacks the transmembrane domain (such as a protein composed of the 1st to 65th amino acid residues, the 1st to 85th amino acid residues, or the 1st to 185th amino acid residues of IL-15Rα, or a peptide containing an amino acid sequence having an identity of not less than 90% to the amino acid sequence), and IL-15.

IL-15/IL-15Rα may have a spacer incorporated between the IL-15 and the IL-15Rα. As the spacer, a peptide composed of usually not more than 300 amino acids, preferably 10 to 100 amino acids, most preferably 20 to 50 amino acids may be used. Specific examples of the spacer include, but are not limited to, a GS linker.

The mbIL15 is not limited as long as it is a transmembrane protein prepared by fusion of IL-15 and IL-15Rα. Specific examples of the mbIL15 include, but are not limited to, a polypeptide having the amino acid sequence of SEQ ID NO: 1. Alternatively, as long as the mbIL15 is capable of binding to an IL-15 receptor to transduce an IL-15 signal into the cell, example of the mbIL15 include, but are not limited to, polypeptides having an amino acid sequence having a homology or an identity of not less than about 90%, preferably not less than about 95%, more preferably not less than about 97%, especially preferably not less than about 98%, most preferably not less than about 99% to the amino acid sequence of SEQ ID NO:1.

The "homology" or "identity" between amino acid sequences means the ratio (%) of identical amino acid residues and similar amino acid residues (in the case of the identity, identical amino acid residues) in all overlapping amino acid residues based on an optimal alignment of two amino acid sequences, which alignment is obtained using a mathematical algorithm known in the art (wherein the algorithm is preferably capable of introducing a gap(s) to one or both of the sequences so as to achieve the optimal alignment). "Similar amino acids" means amino acids having similar physicochemical properties, and examples of the similar amino acids include amino acids classified into the same group, such as those classified into the group of aromatic amino acids (Phe, Trp, and Tyr)), aliphatic amino acids (Ala, Leu, Ile, and Val), polar amino acids (Gln and Asn), basic amino acids (Lys, Arg, and His), acidic amino acid (Glu and Asp), amino acids containing a hydroxy group (Ser and Thr), or amino acids having a small side chain (Gly, Ala, Ser, Thr, and Met). Substitution by such similar amino acids can be predicted not to cause changes in the phenotype of the protein (that is, the substitution is conservative amino acid substitution). Specific examples of conservative amino acid substitution are well known in the art, and described in a variety of literature (see, for example, Bowie et al., Science, 247: 1306-1310 (1990)). The homology or identity of amino acid sequences in the present description can be calculated using the homology calculation algorithm NCBI BLAST (National Center for Biotechnology Information Basic Local Alignment Search Tool) under the following conditions: expectation=10; allow gaps; matrix=BLOSUM62; filtering=OFF.

As described later, the IL-15/IL-15Rα can be expressed by allowing the immunocompetent cell to comprise a polynucleotide encoding the IL-15/IL-15Rα.

### <Decrease in Diacylglycerol Kinase Activity>

Diacylglycerol kinase (DGK) is a lipid kinase that phosphorylates diacylglycerol to convert it into phosphatidic acid. The DGK activity can be measured by a known method. A decrease in the DGK activity may be confirmed based on a decrease in the expression level of DGK by Western blotting, RT-PCR, or the like.

The "decrease in the DGK activity" in the immunocompetent cell may be a decrease in the DGK activity compared to the activity in a control immunocompetent cell. It means, for example, that the DGK activity is not more than 50%, not more than 20%, not more than 10%, not more than 5%, or not more than 1% compared to the activity in a control immunocompetent cell (an immunocompetent cell whose DGK gene is not modified). The DGK activity may be below the detection limit.

As the immunocompetent cell of the present invention, an immunocompetent cell whose DGK activity is originally decreased may be used, or an immunocompetent cell that has been modified to decrease the DGK activity may be used. The decrease in the DGK activity can also be achieved by addition of an inhibitor of the DGK activity.

DGK includes a plurality of kinds of subtypes, and the subtype α and the subtype ζ account for most part of the DGK activity in T cells. Thus, it is preferred to decrease the activities of both the subtype α and the subtype ζ.

Examples of the modification that decreases the DGK activity include introduction of a mutation(s) into the DGK gene on the chromosome, which mutation(s) decrease(s) the activity of DGK, introduction of a mutation(s) into the DGK gene on the chromosome, which mutation(s) decrease(s) the stability of DGK, introduction of a mutation(s) that cause(s) partial or complete deletion of the DGK gene on the chromosome, and introduction of a mutation(s) that decrease(s) the expression level of the DGK gene on the chromosome, such as introduction of a mutation(s) into an expression regulatory region or a translation regulatory region.

Such introduction of a mutation(s) can be carried out by a genome editing technique using CRISPR-Cas9, TALEN, or the like; an RNA interference technique using siRNA, shRNA, miRNA, or the like; a site-specific recombination technique such as a homologous recombination technique or the Cre/Lox P system; or the like. In cases where the Cre/Lox P system is used, for example, introduction of Cre into a DGKα^{-/-}DGKζ^{f/f} cell enables preparation of a cell in which DGKα and DGKζ are double knocked out.

### <Method of Producing Immunocompetent Cell >

In the method of producing an immunocompetent cell of the present invention, an immunocompetent cell having decreased DGK activity, which cell expresses IL-15/IL-15Rα and CAR, is provided.

The immunocompetent cell of the present invention can be obtained by allowing an immunocompetent cell to express CAR, modifying the cell such that the DGK activity is decreased, and allowing the cell to express IL-15/IL-15Rα.

In cases where the DGK activity is already decreased in the immunocompetent cell, the immunocompetent cell of the present invention can be obtained by allowing the immunocompetent cell to express CAR and IL-15/IL-15Rα.

The order of the modification that decreases the DGK activity (i), the expression of CAR (ii), and the expression of IL-15/IL-15Rα (iii) is not limited, and these operations may be carried out in an arbitrary order.

As the immunocompetent cell to be used as the material of the immunocompetent cell of the present invention, an immunocompetent cell such as a cytotoxic T cell isolated from a living body such as peripheral blood mononuclear cells (PBMCs) may be used. In this case, an immunocompetent cell such as a cytotoxic T cell derived from the patient to whom the immunocompetent cell of the present invention is to be administered is preferably used.

The immunocompetent cell to be used as the material of the immunocompetent cell of the present invention may also be an immunocompetent cell obtained by differentiation induction from a pluripotent stem cell. Alternatively, a hematopoietic stem cell or hematopoietic progenitor cell may be induced from a pluripotent stem cell by a known method, and then the immunocompetent cell to be used as the material of the immunocompetent cell of the present invention may be induced from the resulting hematopoietic progenitor cell (for example, WO 2015/199127). In these cases, the modifications (i) to (iii) may be carried out after the differentiation induction of the immunocompetent cell from the pluripotent stem cell; the modifications (i) to (iii) may be carried out at the stage of the pluripotent stem cell; or the modifications (i) to (iii) may be carried out in any stage during the course of the differentiation. In other words, these modifications may be carried out in any order in any stage before the differentiation, during the differentiation, or after the differentiation.

The introduction of the CAR and the IL-15/IL-15Rα into the cell is preferably carried out in the form of polynucleotides encoding these. Each polynucleotide may be either DNA or RNA, or may be a DNA/RNA chimera. The polynucleotide is preferably DNA. As the polynucleotide, a DNA encoding the entire CAR or part thereof may be constructed, for example, by chemically synthesizing a DNA chain based on the amino acid sequence or base sequence of a known CAR, or by linking partially overlapping synthesized oligo-DNA short chains to each other by utilizing the PCR method or Gibson assembly method. The polynucleotide encoding IL-15/IL-15Rα may also be constructed in the same manner.

The polynucleotide may be incorporated in an expression vector. The vector may be either a vector incorporated in the genome of the target cell, or a vector that is not incorporated therein. The expression vector is not limited as long as the vector allows, when it is introduced into the cell, expression of CAR and mbIL15 for a period sufficient for the treatment of a disease. Examples of the expression vector include virus vectors and plasmid vectors. Examples of the virus vectors include retroviral vectors (including lentivirus vectors and pseudotype vectors), adenovirus vectors, adeno-associated virus vectors, herpes virus vectors, Sendai virus vectors, and episomal vectors. A transposon expression system (PiggyBac system) may also be used. Examples of the plasmid vectors include animal cell expression plasmids (such as pa1-11, pXT1, pRc/CMV, pRc/RSV, and pcDNAINeo).

Examples of a promoter used for the vectors include the EF1α promoter, CAG promoter, SRα promoter, SV40 promoter, LTR promoter, CMV (cytomegalovirus) promoter, RSV (Rous sarcoma virus) promoter, MoMuLV (Moloney murine leukemia virus) LTR, HSV-TK (herpes simplex virus thymidine kinase) promoter, TCR Vα gene promoter, and TCR Vβ gene promoter

When desired, the vectors may contain, in addition to the promoter, a transcription or translation regulatory sequence, a ribosome binding site, an enhancer, a replication origin, a poly-A addition signal, a selection marker gene, or the like. Examples of the selection marker gene include the dihydrofolate reductase gene, neomycin resistance gene, and puromycin resistance gene.

The vector that expresses CAR may contain a suicide gene. Such a suicide gene is co-expressed with CAR, and capable of causing cell death when it is activated by an agent such as a drug or an antibody. The suicide gene can be used for improving the safety of the immunocompetent cell that expresses CAR. Examples of the suicide gene include, but are not limited to, inducible caspase 9 (iC9), truncated EGFR (tEGFR), and herpes simplex virus thymidine kinase (HSV-TK). tEGFR is composed of a truncated transmembrane domain and the extracellular domain of EGFR protein. The suicide gene described above may be linked to the polynucleotide encoding CAR through a linker (for example, T2A or IRES).

The method of introducing the polynucleotide or vector into the cell is not limited, and may be a known method. Examples of the method include the electroporation method (Cytotechnology, 3, 133 (1990)), the calcium phosphate method (JP H2-227075 A), the lipofection method (Proc. Natl. Acad. Sci. U.S.A., 84, 7413 (1987)), and the virus infection method. The virus infection method may be, for example, a method in which a packaging cell such as a GP2-293 cell (manufactured by Takara Bio Inc.), Plat-GP cell (manufactured by Cosmo Bio Co., Ltd.), PG13 cell (ATCC CRL-10686), or PA317 cell (ATCC CRL-9078) is transfected with a CAR expression vector (WO 2016/056228) and a packaging plasmid, to prepare a recombinant virus, and then a T cell is infected with the recombinant virus.

The nucleotide encoding CAR and the nucleotide encoding IL-15/IL-15Rα may be incorporated in the genome of a cell by using a known gene editing technique such that they can be expressed under the regulation of appropriate promoters. Examples of the known gene editing technique include techniques using an endonuclease such as zinc finger nuclease, TALEN (Transcription Activator-Like Effector Nuclease), or the CRISPR (Clustered Regularly Interspaced Short Palindromic Repeat)-Cas system.

On the other hand, examples of the method of the modification for decreasing the DGK activity include a method in which one or more kinds of DGK genes on the chromosome are knocked out or knocked down.

The method of knockout of the DGK gene may be a method known in the art, such as a method in which homologous recombination is utilized to replace the entire target gene or part thereof with a drug resistance gene to eliminate the expression of the functional protein, a method utilizing a site-specific recombination reaction such as the Cre/Lox P system, and a genome editing technique utilizing TALEN or CRISPR-Cas9. Methods of knockout of the DGK gene by genome editing are disclosed in, for example, Non-patent Document 2. The knockout of the DGK gene may also be carried out by removing one or more exons of the gene.

Alternatively, the DGK gene may be knocked down. The method of knockdown of the DGK gene may be a method well known in the art, such as the antisense method, in which RNA corresponding to the antisense strand of mRNA is introduced into the cell, or the RNAi method, in which siRNA, shRNA, microRNA, or the like is used. The knockdown efficiency can be confirmed by measuring a change in the mRNA level or the protein expression level. The knockdown of the DGK gene may be carried out by targeting one or more exons of the gene.

### <Method of Induction of Immunocompetent Cell from Pluripotent Stem Cell>

The pluripotent stem cell is not limited as long as it is a stem cell having pluripotency that allows differentiation into many kinds of cells present in a living body, which stem cell also has growth ability and is capable of being induced into an immunocompetent cell. The pluripotent stem cell is preferably derived from a mammal, more preferably derived from a human.

Examples of the pluripotent stem cell include, but are not limited to, embryonic stem (ES) cells, germline stem cells ("GS cells"), embryonic germ cells ("EG cells"), induced pluripotent stem (iPS) cells, pluripotent stem cells derived from cultured fibroblasts or cord blood, and pluripotent cells derived from bone marrow stem cells (Muse cells). The pluripotent stem cell is preferably an iPS cell from the viewpoint of the fact that it is available without destroying an embryo, egg, or the like during the production process. The pluripotent stem cell is more preferably a human iPS cell.

Methods of producing iPS cells are known in the art. These cells can be produced by introducing reprogramming factors into arbitrary somatic cells. Examples of the reprogramming factors herein include genes such as *Oct3*/*4, Sox2, Sox1, Sox3, Sox15, Soxl7, Klf4, Klf2, c-Myc, N-Myc, L-Myc, Nanog, Lin28, Fbx15, ERas, ECAT15-2, Tell, beta-catenin, Lin28b, Sall1, Sall4, Esrrb, Nr5A2, Tbx3,* and *Glis1,* and gene products thereof. These reprogramming factors may be used individually, or may be used in combination. Examples of the combination of the reprogramming factors include those described in WO 2007/069666; WO 2008/118820; WO 2009/007852; WO 2009/032194; WO 2009/058413; WO 2009/057831; WO 2009/075119; WO 2009/079007; WO 2009/091659; WO 2009/101084; WO 2009/101407; WO 2009/102983; WO 2009/114949; WO 2009/117439; WO 2009/126250; WO 2009/126251; WO 2009/126655; WO 2009/157593; WO 2010/009015; WO 2010/033906; WO 2010/033920; WO 2010/042800; WO 2010/050626; WO 2010/056831; WO 2010/068955; WO 2010/098419; WO 2010/102267; WO 2010/111409; WO 2010/111422; WO 2010/115050; WO 2010/124290; WO 2010/147395; WO 2010/147612; Huangfu D, et al. (2008), Nat. Biotechnol., 26: 795-797; Shi Y, et al. (2008), Cell Stem Cell, 2: 525-528; Eminli S, et al. (2008), Stem Cells. 26:2467-2474; Huangfu D, et al. (2008), Nat. Biotechnol. 26:1269-1275; Shi Y, et al. (2008), Cell Stem Cell, 3, 568-574; Zhao Y, et al. (2008), Cell Stem Cell, 3:475-479; Marson A, (2008), Cell Stem Cell, 3, 132-135; Feng B, et al. (2009), Nat. Cell Biol. 11:197-203; R.L. Judson et al., (2009), Nat. Biotechnol., 27:459-461; Lyssiotis CA, et al. (2009), Proc Natl Acad Sci USA. 106:8912-8917; Kim JB, et al. (2009), Nature. 461:649-643; Ichida JK, et al. (2009), Cell Stem Cell. 5:491-503; Heng JC, et al. (2010), Cell Stem Cell. 6:167-74; Han J, et al. (2010), Nature. 463:1096-100; Mali P, et al. (2010), Stem Cells. 28:713-720; and Maekawa M, et al. (2011), Nature. 474:225-9.

Examples of the somatic cells used for the preparation of the iPS cells include, but are not limited to, any of fetal somatic cells, neonatal somatic cells, and mature, healthy or diseased somatic cells, and any of primary cultured cells, subcultured cells, and established cell lines. Specific examples of the somatic cells include: (1) tissue stem cells (somatic stem cells) such as neural stem cells, hematopoietic stem cells, mesenchymal stem cells, and dental pulp stem cells; (2) tissue progenitor cells; and (3) differentiated cells such as blood cells (peripheral blood cells, cord blood cells, and the like), lymphocytes, epithelial cells, endothelial cells, muscle cells, fibroblasts (skin cells and the like), hair cells, hepatic cells, gastric mucosal cells, enterocytes, spleen cells, pancreatic cells (pancreatic exocrine cells and the like), brain cells, lung cells, kidney cells, and adipocytes. For the induction of the immunocompetent cell, an immunocompetent cell is preferably used as the somatic cell. However, use of a non-immunocompetent cell also enables the production of the immunocompetent cell from the iPS cell.

In the present invention, the mammalian individual from which the somatic cells are collected is not limited. The mammalian individual is preferably a human. In cases where the immunocompetent cell of the present invention is administered to a subject, the somatic cell to be used as the origin of the iPS cell is preferably isolated from the subject, from the viewpoint of simply matching the type of the human leukocyte antigen (HLA) with that of the subject.

The method of the differentiation induction from the pluripotent stem cell into the cytotoxic T cell is not limited, and a known method may be used therefor. Examples of the method include a method in which a hematopoietic progenitor cell is induced from the pluripotent stem cell, and then a CD4/CD8 double positive cell is induced from the hematopoietic progenitor cell, followed by inducing a CD8-positive cell from the CD4/CD8 double positive cell. Specific examples of the method of the differentiation induction from the pluripotent stem cell into the cytotoxic T cell include the methods described in Patent Documents 1 to 3, but the method is not limited as long as the cytotoxic T cell can be obtained.

The immunocompetent cell such as a cytotoxic T cell obtained by the differentiation induction from the pluripotent stem cell may be used as it is, or may be used after sorting using a marker such as CD8β or CD5.

Specific examples of the method of differentiation induction from the pluripotent stem cell into an immunocompetent cell other than a cytotoxic T cell include the methods described in the following documents.
CD4 helper T cell ... WO 2017/065288, WO 2018/168829
γδT cell ... WO 2020/013315
NK cell ... Blood (2016) 128(22): 1345, WO 2013/163171, WO 2010/099539
NKT cell ... Stem cell Rep. 2016 Feb 9; 6(2): 213-227.
doi:10.1016/j.stemcr.2016.01.005., Stem Cells. 2016 Dec; 34(12): 2852-2860
Monocyte/macrophage ... WO 2015/199127
Lymphoid cells such as B cells ... JP 2006-141356 A
Antigen-presenting cells such as dendritic cells ... WO 2012/115276, Clin Cancer
Res. 2008 Oct 1; 14(19): 6207-6217
Granulocytes such as neutrophils, eosinophils, basophils, and mast cells ... WO 2015/199127, JP 2006-141356 A

### <Use of Immunocompetent Cell >

The present invention provides a pharmaceutical composition comprising the immunocompetent cell of the present invention.

Since the immunocompetent cell of the present invention is capable of showing cytotoxic activity against cancer cells, virus-infected cells, and the like, a pharmaceutical composition comprising the cytotoxic T cell of the present invention can be used for treatment of infections (such as chronic infections), autoimmune disorders, and the like.

The pharmaceutical composition of the present invention can be administered to a mammal (such as a mouse, rat, hamster, rabbit, cat, dog, cow, sheep, monkey or human), preferably a human. Thus, in one mode of the present invention, a pharmaceutical composition comprising the immunocompetent cell of the present invention for use in treatment of cancer is provided. Further, a method for treating cancer comprising administering a therapeutically effective amount of the immunocompetent cell of the present invention or a pharmaceutical composition containing the immunocompetent cell to a subject in need of the treatment is provided.

Specific examples of the cancer include, but are not limited to, liver cancers (such as hepatocellular carcinoma), ovarian cancers (such as ovarian clear cell adenocarcinoma), childhood cancers, lung cancers (such as squamous cell carcinoma and lung small cell carcinoma), testicular cancers (such as nonseminoma germ cell tumor), soft tissue tumors (such as liposarcoma and malignant fibrous histiocytoma), uterine cancers (such as cervical intraepithelial neoplasia and cervical squamous cell carcinoma), melanoma, adrenal tumors (such as adrenal adenoma), neurogenic tumors (such as schwannoma), gastric cancers (such as gastric adenocarcinoma), renal cancers (such as Grawitz tumor), breast cancers (such as invasive lobular carcinoma and mucinous carcinoma), thyroid cancers (such as medullary carcinoma), laryngeal cancers (such as squamous cell carcinoma), and bladder cancers (such as invasive transitional cell carcinoma).

The immunocompetent cell of the present invention may be cultured and/or stimulated using an appropriate medium and/or a stimulatory molecule before being administered to the subject. Examples of the stimulatory molecule include, but are not limited to, cytokines, appropriate proteins, and other components. Examples of the cytokines include IL-2, IL-7, IL-12, IL-15, and IFN-γ. IL-2 may be preferably used. The concentration of IL-2 in the medium is not limited, and is preferably 0.01 U/ml to 1×10⁵ U/ml, more preferably 1 U/ml to 1×10⁴ U/ml. Examples of the appropriate proteins include CD3 ligand, CD28 ligand, and anti-IL-4 antibodies. Further, a lymphocyte-stimulating factor such as lectin may be added. Further, serum or plasma may be added to the medium. These may be added in arbitrary amounts to the medium. Examples of the amounts include 0% by volume to 20% by volume. The serum or plasma may be used in different amounts in different culture stages. For example, the serum or plasma concentration may be decreased stepwise. The origin of the serum or plasma may be either autologous or allogeneic. From the viewpoint of safety, it is preferably autologous.

The pharmaceutical composition of the present invention is preferably used by parenteral administration to the subject. Examples of the method of the parenteral administration include intravenous, intraarterial, intramuscular, intraperitoneal, or subcutaneous administration, and direct administration to the affected area. The dose is appropriately selected depending on the conditions, body weight, age, and the like of the subject. The administration is carried out at a dose of usually 1×10⁶ to 1×10¹⁰ cells, preferably 1×10⁷ to 1×10⁹ cells, more preferably 5×10⁷ to 5×10⁸ cells each time in terms of the cell number per subject having a body weight of 60 kg. The pharmaceutical composition may be administered once, or may be administered a plurality of times. The pharmaceutical composition of the present invention may be prepared in a known form suitable for parenteral administration, such as an injection or infusion. When appropriate, the pharmaceutical composition of the present invention may contain a pharmaceutically acceptable excipient or carrier. The pharmaceutical composition of the present invention may contain physiological saline, phosphate-buffered saline (PBS), a medium, or the like for stably maintaining the cells. Examples of the medium include, but are not limited to, media such as RPMI, AIM-V, and X-VIVO10. Further, a pharmaceutically acceptable carrier (such as human serum albumin), a preservative, and/or the like may be added to the pharmaceutical, for the purpose of stabilization.

Since the immunocompetent cell of the present invention is capable of killing cells expressing target antigens such as the above-described tumor antigens, the immunocompetent cell may be used as a cytocidal agent against cells expressing the antigens (such as cancer cells, cancer stem cells, and tumor cells). Such a cytocidal agent may be prepared and used in the same manner as the pharmaceutical described above.

The immunocompetent cell of the present invention may be used in combination with other anticancer drugs. Examples of the other anticancer drugs include alkylating agents such as cyclophosphamide, bendamustine, ifosfamide, and dacarbazine; antimetabolites such as pentostatin, fludarabine, cladribine, methotrexate, 5-fluorouracil, 6-mercaptopurine, and enocitabine; molecular targeted drugs such as rituximab, cetuximab, and trastuzumab; kinase inhibitors such as imatinib, gefitinib, erlotinib, afatinib, dasatinib, sunitinib, and trametinib; proteasome inhibitors such as bortezomib; calcineurin inhibitors such as cyclosporine and tacrolimus; anticancer antibiotics such as idarubicin and doxorubicin mitomycin C; plant alkaloids such as irinotecan and etoposide; platinum formulations such as cisplatin, oxaliplatin and carboplatin; hormone therapy drugs such as tamoxifen and bicalutamide; and immunoregulatory drugs such as interferon, nivolumab, and pembrolizumab.

Examples of a method in which the immunocompetent cell of the present invention is used in combination with another anticancer drug include a method in which treatment with the other anticancer drug is carried out followed by using the immunocompetent cell of the present invention, a method in which the immunocompetent cell of the present invention and the other anticancer drug are used at the same time, and a method in which treatment with the immunocompetent cell of the present invention is carried out followed by using the other anticancer drug. In cases where the immunocompetent cell of the present invention is used in combination with another anticancer drug, an improved therapeutic effect on cancer can be obtained, and, by decreasing the number of times of administration or the dose of each of the immunocompetent cell and the anticancer drug, side effects caused by each of these can be reduced.

Further, in one mode, the present invention includes use of the immunocompetent cell of the present invention in the manufacture of a cancer therapeutic agent in accordance with the pharmaceutical composition comprising the immunocompetent cell of the present invention. The cancer therapeutic agent can be produced by a method known per se. For example, similarly to the method of preparing the pharmaceutical of the present invention, the cancer therapeutic agent may be produced in a known form suitable for parenteral administration, such as an injection or infusion.

### EXAMPLES

The present invention is described below concretely by way of Examples. However, the present invention is not limited to the modes in the following Examples.

### <Experimental Procedure>

### Preparation of CAR-Introduced iPS Cells

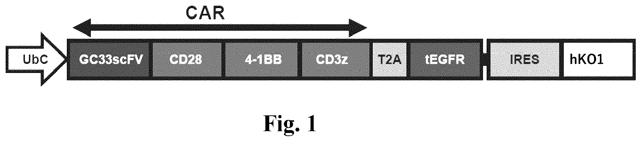

GC33-CD28-41BB-T2AEGFR, which was designed by Prof. Tamada, Yamaguchi University, and prepared by Genscript, was used as the CAR. The CAR has a CD8 transmembrane domain as the transmembrane domain. Introduction of the CAR gene into iPS cells was carried out using the lentivirus vector CS-UbC-RfA-IRES-hKO1. This vector expresses a humanized Kusabira-Orange 1 (hKO1) fluorescent protein gene linked to an IRES sequence, under the regulation of the human ubiquitin C (UbC) promoter. As iPS cells, the TKT3v 1-7 strain, which was established using the method described in Nishimura T, et al., Cell Stem Cell. 12(1):114-126, 2013 from human CD3-positive T cells isolated with informed consent, was used. The lentivirus vector CS-UbC-RfA-IRES-hKO1, in which the GC33-CD28-41BB-T2A EGFR gene was incorporated (a schematic diagram of part of the vector is shown in Fig. 1), was introduced into the TKT3v1-7 strain, and iPS cells expressing hKO1 (CAR-iPSC) were selected.

### Preparation of iPSC Deficient for DGK by CRISPR-Cas9

Single guide RNAs specific to exon 7 of DGKα (diacylglycerol kinase alpha) and exon 2 of DGKζ (diacylglycerol kinase zeta) were designed (SEQ ID NOs: 2 and 3, respectively). For construction of custom sgRNA expression vectors, two kinds of oligonucleotides containing an sgRNA target site and a universal reverse primer (SEQ ID NOs:4 and 5 for DGKα deficiency; SEQ ID NOs:6 and 5 for DGKζ deficiency) were amplified by PCR, and cloned into the BamHI-EcoRI site of the pHL-H1-ccdB-mEFα-RiH vector (addgene #60601), which uses the H1 promoter for sgRNA expression. A plasmid that expresses a nuclease (5 µg of pHL-EF1α-SphcCas9-iP) and 5 µg of a guide RNA expression vector (for DGK deficiency) were introduced into 1×10⁶ CAR-iPSCs by the electroporation method using an NEPA 21 electroporator (electroporation pulse: pulse voltage, 175 V; pulse width, 2.5 ms; number of times of pulse application, 2; Negagene). An iPSC clone in which nonsense mutations are introduced to both DGKα and DGKζ was selected (DGK-dKO CAR-iPSC).

### T Cell Differentiation from CAR-iPSCs

For allowing iPSCs to differentiate into T cells, a reported protocol (Cell Stem Cell1-9 (2018). doi:10.1016/j.stem.2018.10.005) was carried out with modifications. First, CAR-iPSCs were allowed to differentiate into hematopoietic progenitor cells through SAC formation on feeder cells or through embryoid body formation under feeder-free conditions. In the method in which SACs were formed, iPSC clusters were plated on C3H10T1/2 feeder cells, and cultured in EB medium supplemented with rhVEGF (R&D). On Day 7, rhSCF (Peprotech) and rhFlt-3L (Peprotech) were added to the culture liquid. In the method in which feeder-free embryoid bodies were formed, undifferentiated CAR-iPSC colonies were treated with TrypLE select (Gibco) for 8 minutes, and then plated on a low-adhesive plate, to allow formation of embryoid bodies in Stemfit AK03N supplemented with 10µM Y-27632. After overnight incubation, embryoid body formation was promoted. The embryoid bodies were collected, and plated on EB medium (StemPro-34 (Invitrogen), 2 mM l-glutamine, 400 µM monothioglycerol, 50 µg/ml ascorbic acid 2-phosphate, and insulin-transferrin-selenium medium supplement), followed by performing culture in the presence of 40 ng/ml hBMP-4, 10 ng/ml hbFGF, and 50 ng/ml VEGF. On Day 4, the embryoid bodies were cultured together with a cocktail of hematopoietic cytokines (50 ng/ml hSCF, 20 ng/ml hFlt3L, 20 ng/ml hIL-3, and 30 ng/ml TPO). On Day 8 to 14 of the culture, differentiated cells were plated on Delta-like-1-expressing OP9 (OP9-DL1) feeder cells or on a plate coated with FcDLL4, followed by performing culture in the presence of a cocktail (10 ng/ml hFlt3L and 5 ng/ml IL-7) of cytokines for growing T-lineage cells (T-lineage cytokines). On Days 21 to 28 of the culture, the hematopoietic cells differentiated into CD3+ T-lineage cells. The T-lineage cells were collected, and stimulated with 500 ng/ml OKT3 (Miltenyi Biotec) in a-MEM (SIGMA) supplemented with 15% fetal bovine serum (Thermo), 2 mM L-glutamine (Thermo), 100 U/ml penicillin (Thermo), and 1G00 ng/ml streptomycin (Thermo), in the presence of 10 ng/ml rhIL-7 and 10 nM dexamethasone (Dexart, Japan, Fuji Pharma Co., Ltd.).

From the obtained cell population, CD5/CD8β double positive T cells were sorted, and these cells were used as iCAR-T_{CTL} in the following experiment.
iCAR-T_{CTL} was mixed with irradiated PBMCs, and subjected to expansion culture, followed by co-culture in alpha-MEM medium in the presence of 10 ng/ml IL-7, 5 ng/ml IL-15 (Peprotech), and 2 µg/ml PHA (SIGMA).

DGK-dKO CAR-iPSC was allowed to differentiate by the same method, and CD5/CD8β double positive T cells were sorted. These cells were used as DGK-dKO iCAR-T_{CTL} in the following experiment.

### Flow Cytometry Analysis

A stained cell sample was analyzed by LSR or FACS AriaII Flow Cytometer (BD Biosciences), and data processing was carried out using FlowJo (Tree Star). For phenotype analysis of T cells, the following antibodies were used: APC-cy7-CD3 (clone UCHT1, BioLegend), eFluor 450-CD3 (clone UCHT1, eBioscience), BV421-CD4 (clone OKT4, BioLegend), BV510-CD4 (clone OKT4, BioLegend), FITC-CD5 (clone UCHT2, eBioscience), PE-Cy7-CD5 (clone UCHT2, eBioscience), CD7-APC (clone CD7-6B7, BioLegend), PerCPcy5.5-CD8 (clone SK1, BioLegend), APC-CD8β (clone 2ST8.5H7, BD), PE-Cy7-CD8β (clone SIDI8BEE, eBioscience), BV510-CD45RA (clone HI100, BioLegend), APC-cy7-CD45RO (clone UCHL1, BioLegend), FITC-CD62L (clone DREG-56, BioLegend), APC-CD197 (CCR7) (clone G043H7, BioLegend), BV421-CD28 (clone CD28.2, BioLegend), APC-CD27 (clone O323, BioLegend), BV421-CD279 (PD-1) (clone EH12.2H7, BioLegend), and PerCP-eFluor 710-TIGIT (clone MBSA43, eBioscience).

### Growth Assay by ³H-Thymidine Incorporation

The growth of T cells was measured using a ³H-thymidine incorporation assay. Co-culture of 1×10⁵ effector cells with irradiated 1×10⁴ target cells was carried out for 3 days. After pulse labeling with ³H, 3H-thymidine was measured 16 hours later using a β counter.

### Introduction of CAR into Primary T-Cells, and Preparation of DGK-Deficient Primary T-Cells

To obtain primary T cells, blood cells were isolated from blood collected from a healthy donor, and T cells were isolated and stimulated using Thermo Fisher Dynabeads Human T-Activator CD3/CD28. Three days after the stimulation, CAR was introduced into the primary T cells using a lentivirus. Cells into which the CAR was introduced were collected by flow cytometry using CAR-positivity and CD8-positivity as indices (pCAR-T_{CTL} cells).

### Primary T cells with partial deficiency of DGK were prepared by the following procedure (DGK-pKO pCAR-T_{CTL} cells).

The primary T cells were stimulated using Dynabeads Human T Activator anti-CD3/28 (Thermo Scientific). Four days after the stimulation, electroporation was carried out using an Amaxa P3 Primary Cell Kit and 4D-Nucleofecter (Lonza). After incubating 10 µg of genetically modified *S. pyogenes* Cas9 (Thermo Fisher Scientific) and 1.25 µg of chemically synthesized sgRNAs for DGKα- and ζ-deficiencies (for the α deficiency, oligonucleotides of SEQ ID NOs:7 and 8 were used, and, for the ζ deficiency, oligonucleotides of SEQ ID NOs:9 and 8 were used in the synthesis) for 20 minutes, electroporation was carried out to prepare a Cas9-gRNA RNP complex. A total of 5×10⁵ cells were resuspended, and then P3 buffer was added to the preincubated Cas9-gRNARNP complex. Using the program EO-115, nucleofection was carried out. One week after the electroporation, the cells were collected, and genomic DNA was isolated. Analysis was carried out using Tracking of Indels by Decomposition (TIDE) (Nucleic Acids Res. 42, (2014)), to calculate the indel frequency.

### Preparation of CAR-T Cells in Which mbIL-15 Is Introduced by Gene Transfer

RD18 cells capable of producing a retroviral vector encoding mbIL15-T2A-NGFR were kindly provided by Dr. Kazuhide Nakayama, Takeda Pharmaceutical Company Limited. The retroviral vector comprises a base sequence encoding a polypeptide (SEQ ID NO: 1) containing, in the order from the N-terminus to the C-terminus, (i) a leader sequence of human IL-2 (23 amino acids), (ii) a C-terminal sequence of human IL-15 (114 amino acids), (iii) a GS linker (24 amino acids), and (iv) a C-terminal sequence of human IL-15Rα (239 amino acids). Each type of CAR-T_{CTL} cells were stimulated using Dynabeads Human T Activator anti-CD3/28 (Thermo Scientific). Three days after the stimulation, the cells were subjected to gene transfer using the retroviral vector encoding mbIL15-T2A-NGFR. NGFR-positive cells were purified by FACS.

The cells obtained by introducing mbIL-15 into the iCAR-T_{CTL} cells or the DGK-dKO iCAR-T_{CTL} cells were named iCAR-T_{CTL} mbIL cells (also referred to as "iCAR-T_{CTL} mbIL15tg" or "iCAR-T_{CTL} mbIL15" in the figures) and DGK-dKO iCAR-T_{CTL} mbIL cells (also referred to as "DGK-dKO iCAR-T_{CTL} mbIL15tg", "DGK-dKO iCAR-T_{CTL} mbIL15", or "DGKdKO iCAR-T_{CTL} mbIL15tg" in the figures), respectively.

The cells obtained by introducing mbIL-15 into the pCAR-T_{CTL} cells or the DGK-pKO pCAR-T_{CTL} cells were named pCAR-T_{CTL} mbIL cells (also referred to as "pCAR-T_{CTL} mbIL15tg" or "pCAR-T_{CTL} mbIL15" in the figures) and DGK-pKO pCAR-T_{CTL} mbIL cells (also referred to as "DGK-pKO pCAR-T_{CTL} mbIL15tg", "DGK-pKO pCAR-T_{CTL} mbIL15", or "DGKpKO pCAR-T_{CTL} mbIL15tg" in the figures), respectively.

### Bioluminescence Imaging

Detection of bioluminescence was carried out using the Xenogen IVIS Imaging System (Xenogen). VivoGlo (trademark) luciferin (3 mg/mouse; Promega) was intraperitoneally injected, and imaging was carried out 10 to 15 minutes thereafter.

### In Vivo Tumor Models

### Peritoneal Dissemination Animal Model #1

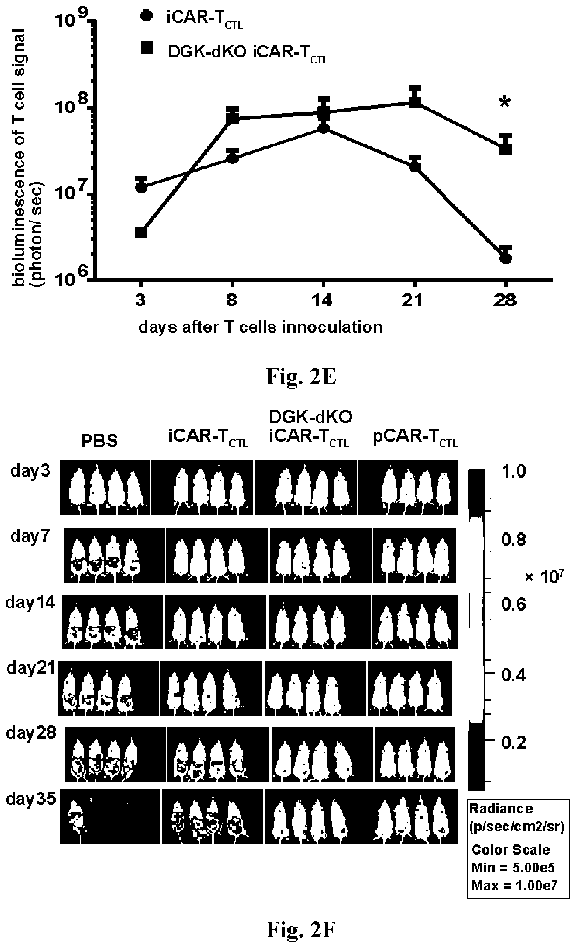

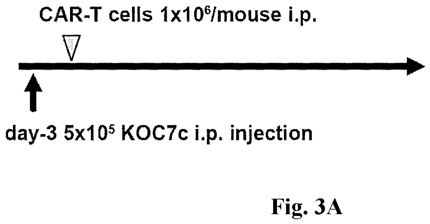

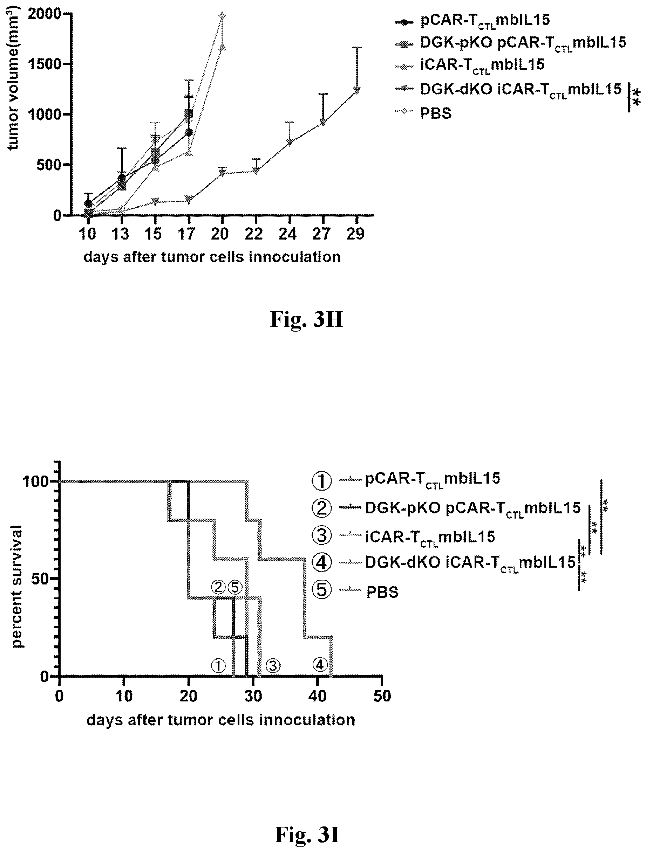

A total of 5×10⁵ ovarian cancer cells KOC7c in which the luciferase gene is introduced were intraperitoneally injected into NSG mice, and then each type of CAR-T_{CTL} cells in an amount of 5×10⁶ cells were intraperitoneally injected on Days 3, 7, 10, and 14 (Figs. 2F to H), or CAR-T cells in an amount of 1×10⁶ cells were intraperitoneally injected on Day 3 (Figs. 3A to E). The amount of tumor was measured by in vivo bioluminescence imaging (IVIS 100 Imaging System, Caliper).

### Analysis of Tumor-Infiltrating CAR-T Cell Model

A total of 5×10⁶ SK-Hep-1 cells in which GPC3 was introduced by gene transfer were subcutaneously injected into the left abdomen of NSG mice. On Day 0, each type of CAR-T_{CTL} cells were intravenously injected. On Day 7, the mice were sacrificed, and subcutaneous tumors were collected. Each tumor was divided into two pieces. One of these divided pieces was thoroughly minced with a razor blade, and gently homogenized using MACS Dissociator. Subsequently, the homogenized product was filtered through a 70-mm nylon mesh cell strainer. The single-cell suspension was subjected to staining with a fluorescent dye-labeled antibody as described above. The other divided piece was subjected to preparation for histopathological analysis.

### Subcutaneous Tumor Animal Model #1

On Day 14, liver cancer SK-Hep-1 cells in which GPC3 was introduced by gene transfer, in an amount of 5×10⁶ cells, were subcutaneously injected into the left abdomen of NSG mice. SK-Hep-1 cells that had not been subjected to the gene transfer, in an amount of 5×10⁶ cells, were subcutaneously injected into the right abdomen of the NSG mice. On Day 0, each type of CAR-T_{CTL} cells were intravenously injected. The luciferase activity of each type of CAR-T_{CTL} cells was measured using in vivo bioluminescence imaging.

### Subcutaneous Tumor Animal Model #2

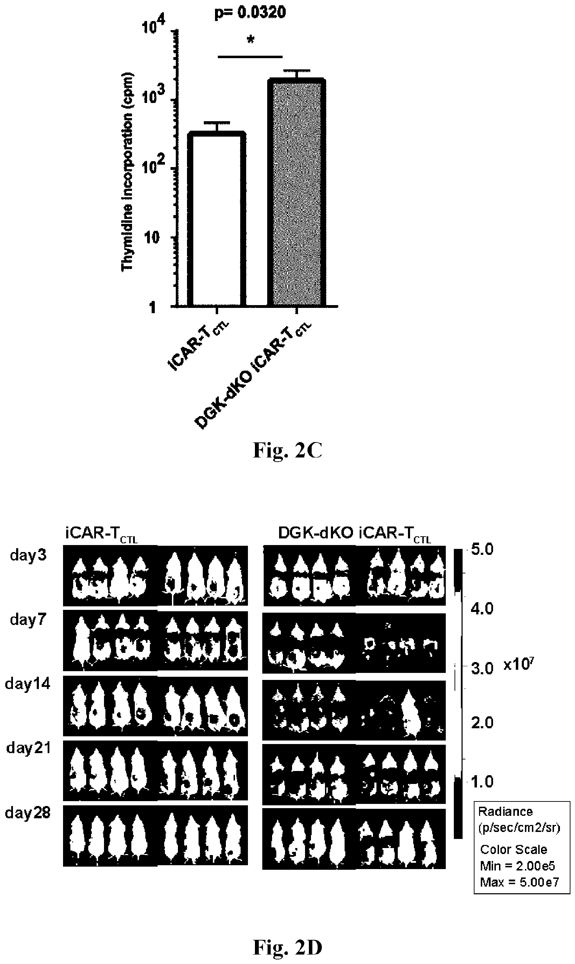

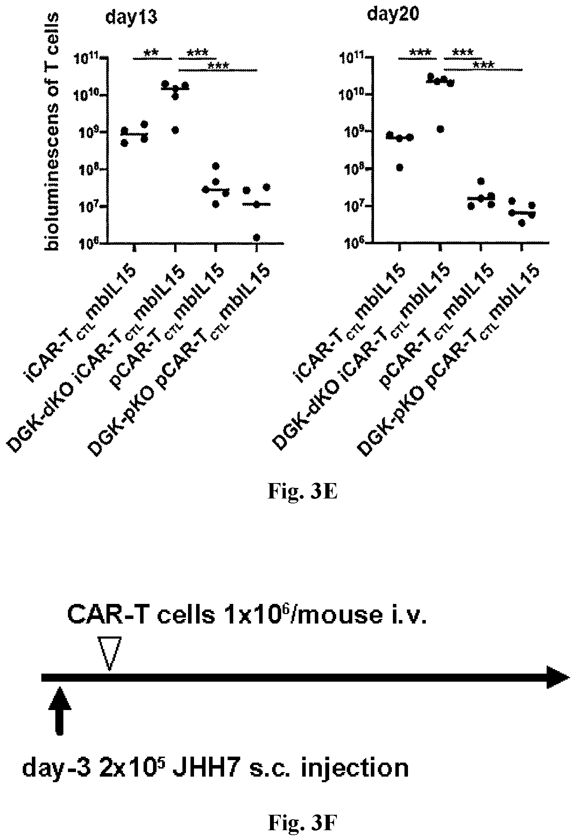

A total of 2×10⁵ JHH7 cells were subcutaneously injected, and each type of CAR-T_{CTL} cells in an amount of 1×10⁷ cells were intravenously injected on Day 3 and Day 10 (Figs. 2D and E), or CAR-T cells in an amount of 1×10⁶ cells were intravenously injected on Day 3 (Figs. 3F to J). The luciferase activity of the T cells was measured using in vivo bioluminescence imaging. The size of the tumor was measured using a vernier caliper, and the tumor volume was calculated according to the following equation: V=LW²/2 (wherein L represents the length (major axis), and W represents the width (minor axis))

### Subcutaneous Tumor Animal Model #3

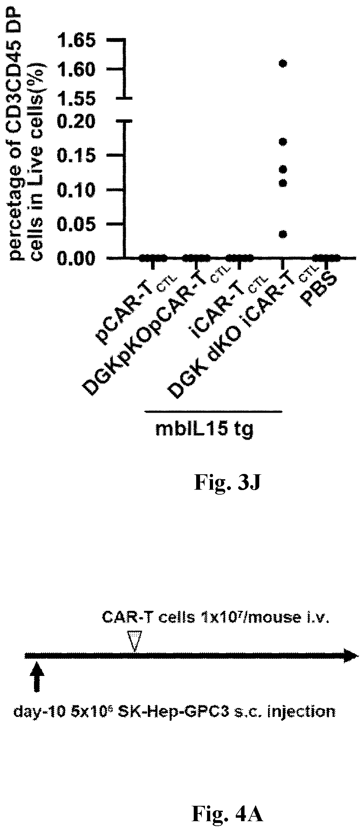

A total of 5×10⁵ SK-Hep-GPC cells were subcutaneously injected, and each type of CAR-T_{CTL} cells in an amount of 1×10⁷ cells were intravenously injected on Day 10 (Figs. 4A to C). The size of the tumor was measured using a vernier caliper, and the tumor volume was calculated according to the following equation: V=LW²/2 (wherein L represents the length (major axis), and W represents the width (minor axis))

### Peritoneal Dissemination Animal Model #2

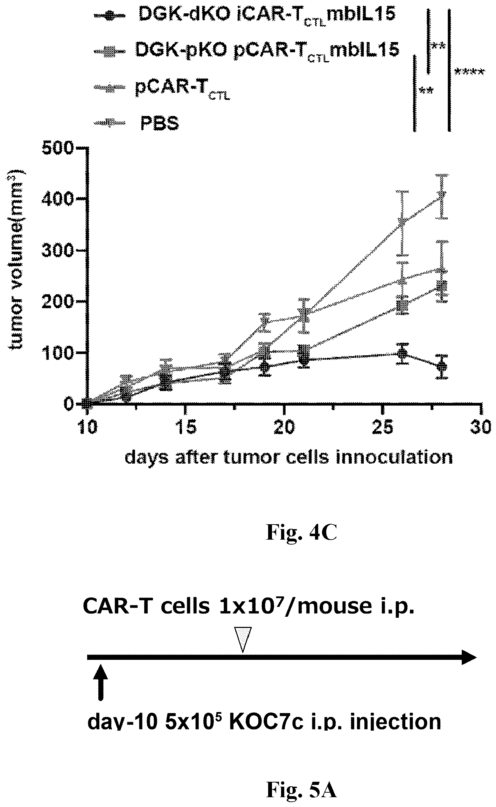

A total of 5×10⁵ KOC7c cells in which the luciferase gene is introduced were intraperitoneally injected into NSG mice, and then each type of CAR-T_{CTL} cells in an amount of 1×10⁷ cells were intraperitoneally injected once on Day 10 (Figs. 5A to D). The luciferase activity of the T cells was measured using in vivo bioluminescence imaging.

### Statistics

All data are expressed as mean ± SEM. All statistic analyses were carried out using Prism (GraphPad software). For comparison among a plurality of groups, ANOVA and the Turkey multiple comparison test were carried out. For comparison between two groups of parameter data, the two-sided Student's t-test was carried out. A p-value of of less than 0.05 was regarded as significant.

### <Results>

As a result of the marker analysis and marker-based cell sorting of cytotoxic T cells obtained by differentiation induction from CAR-iPSCs, CD5-positive/CD8β-positive cells were found to have a profile similar to that of cytotoxic T cells in a living body regarding an expression profile of genes related to naiveness and trafficking properties of T cells, indicating that the above cells have excellent trafficking properties and cytotoxicity against cancer cells. In view of this, cytotoxic T cells obtained by differentiation induction from CAR-iPSCs were sorted using CDS-positivity and CD8β-positivity as indices, and the obtained cells were used as iCAR-T_{CTL} cells.

Based on the result of the in vivo bioluminescence imaging of the subcutaneous tumor animal model #1, the site of transplantation of the liver cancer SK-Hep-1 cells in which GPC3 was introduced by gene transfer (SK-Hep-GPC3) was found to show a slow selective increase in the luminescence intensity from Day 3 to Day 7 in the mice to which iCAR-T_{CTL} was injected and the mice to which pCAR-T_{CTL} was injected. In the site of transplantation of the SK-Hep-1 cells (SK-Hep) that had not been subjected to the gene transfer, no accumulation of these cells was found.

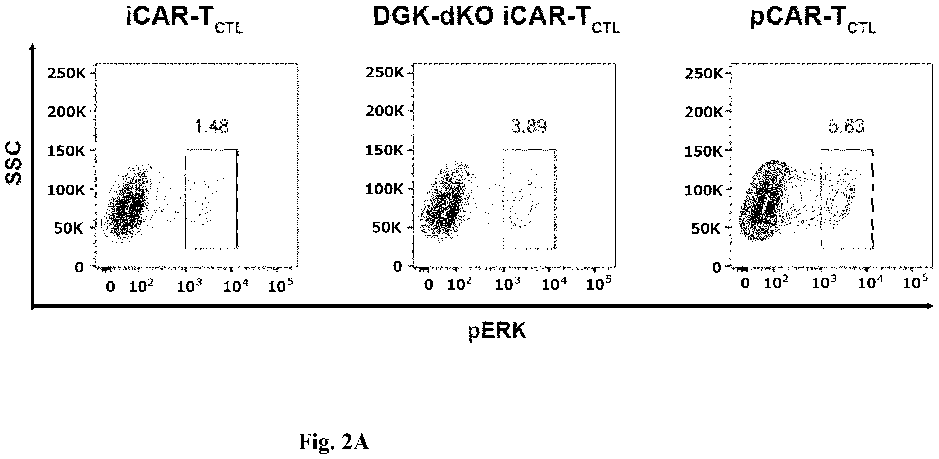

However, when a heterologous tumor transplantation model with peritoneal dissemination was treated with iCAR-T_{CTL}, the survival rate was lower than that in the case of pCAR-T_{CTL}. As a result of analysis of phosphorylation based on flow cytometry (Phosflow), one possible cause of the above result was found to be a decrease in the MEK/ERK signal transduction mediated by CAR (Fig. 2A, left panel). Binding of CAR to the target molecule causes CD3ζ of phosphorylated CAR to produce diacylglycerol (DAG) from phosphatidylinositol 4,5-bisphosphate (PI(4,5)P₂), leading to activation of the low-molecular-weight GTP-binding protein RAS, and ERK1, ERK2, and AP1, which are downstream molecules thereof. In the case of co-culture with SK-Hep-GPC3, phosphorylation of ERK1/2 in iCAR-T_{CTL} was lower than that in the case of pCAR-T_{CTL} (Fig. 2A)

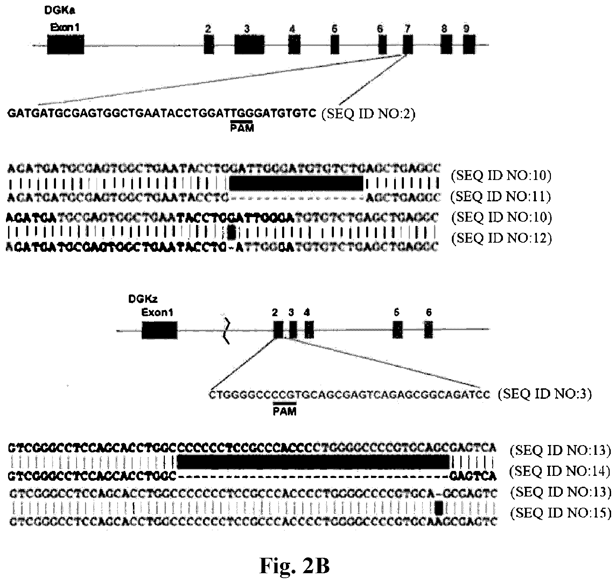

In view of this, for enhancing signal transduction intensity of T cells, the inventors focused on DAG metabolic regulation. Phosphorylation of CD3ζ through TCR or CAR causes DAG to recruit and activate RasGRP1 (Ras guanyl nucleotide-releasing protein 1), to cause activation of the MEK/ERK pathway. DGKα and DGKζ are two major DGK isoforms found in T cells. It is known that degradation of DAG leads to decreased MEK/ERK signal transduction ((Sci. Signal. 8, re6-re6 (2015)). Further, inhibition of DGK promotes the activation of AP-1 and NF-xB through the RAS/ERK pathway (Nat. Immunol. 7, 1166-1173 (2006)). Based on this knowledge related to enhancement of T cell signal transduction, both DGKα and DGKζ were deleted using CRISPR-Cas9 in the iPSC stage (DGK-dKO iCAR, Fig. 2B), and the resulting iPSCs were allowed to differentiate into iCAR-T_{CTL} cells. The obtained cells were named DGK-dKO iCAR-T_{CTL}. As a result of Phosflow measurement of DGK-dKO iCAR-T_{CTL}, it was shown that stimulation of the cells with the target antigen causes partial recovery of phosphorylation of ERK1/2 (Fig. 2A, middle panel). By the recovery of phosphorylation of ERK, the growth ability of DGK-dKO iCAR-T_{CTL} that responds to SK-Hep-GPC3 was remarkably improved compared to iCAR-T_{CTL} having no deficiency of DGK (Fig. 2C).

Subsequently, in order to confirm whether or not deficiency of DGK affects the in vivo function of iCAR-T_{CTL}, the accumulation performance and the survival performance of DGK-dKO iCAR-T_{CTL} were evaluated using a subcutaneous tumor heterologous transplantation model. From the result of an in vivo imaging assay, it was found that DGK-dKO iCAR-T_{CTL} accumulates better and survives longer in a tumor site compared to iCAR-T_{CTL} (Figs. 2D and E).

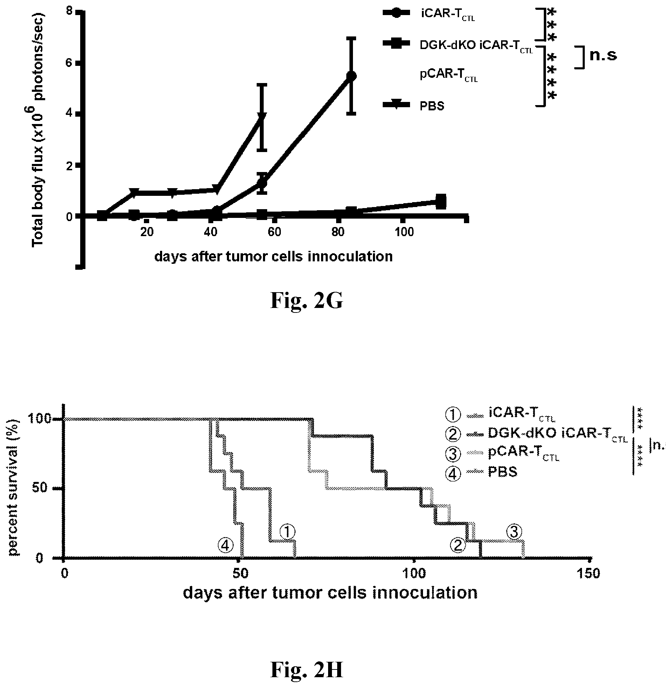

Subsequently, in order to evaluate the effector function in tumor-inoculated NSG mice, a KOC7c peritoneal dissemination model was used. As a result, DGK-dKO iCAR-T_{CTL} showed stronger suppression of the tumor growth compared to iCAR-T_{CTL} (Figs. 2F and G), and prolonged the survival time of the mice to about the same level as that of the pCAR-T_{CTL} treatment group (Fig. 2H).

Subsequently, in order to design DGK dKO iCAR-T_{CTL} showing a higher cancer therapeutic effect than pCAR-T_{CTL}, the inventors focused on the apoptosis-suppressing effect and the growth effect of the transmembrane-type IL-15/IL-15Rα (mbIL15) gene. As a result of introduction of the mbIL15 gene into pCAR-T_{CTL}, DGK-pKO pCAR-T_{CTL}, iCAR-T_{CTL}, and DGK-dKO iCAR-T_{CTL} using a retroviral vector, and investigation of the growth ability of the cells, the growth ability of iCAR-T_{CTL} through the target in vitro was remarkably improved due to the introduction of the mbIL15 gene irrespective of the presence or absence of DGK gene modification.

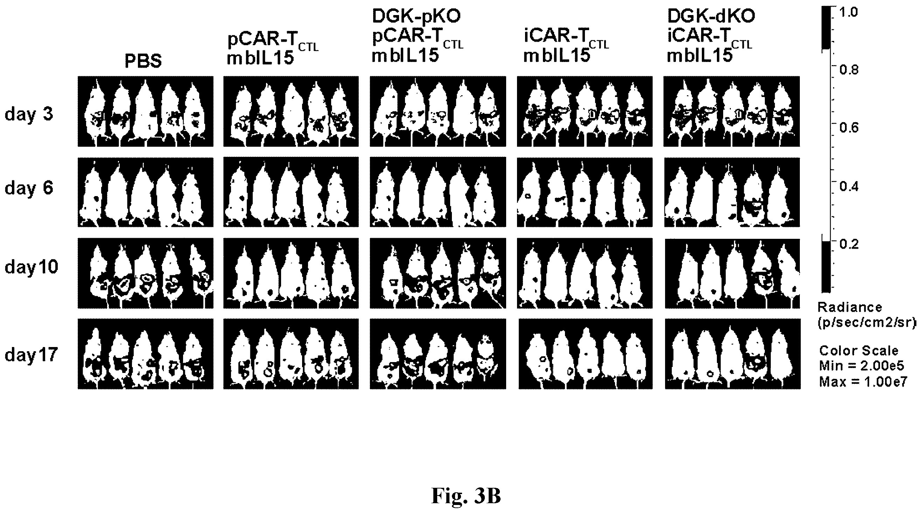

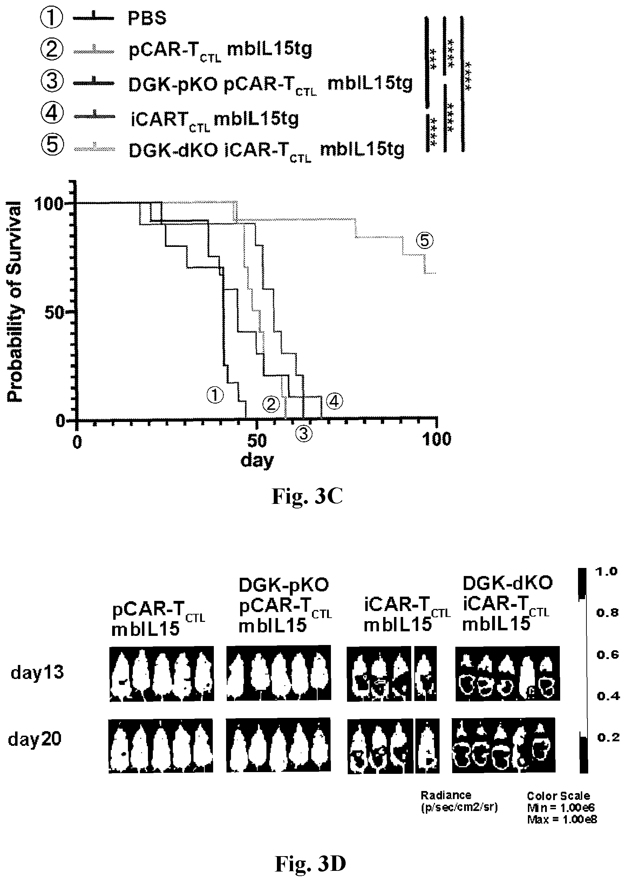

In order to investigate whether or not the in vivo anti-tumor effect can be improved by treatment with DGK-knockout CAR-T cells, each type of CAR-T_{CTL} cells in an amount of 1×10⁶ cells were intraperitoneally injected to peritoneal dissemination mice to which KOC7c had been preliminarily inoculated (Fig. 3A). In this heterologous transplantation animal model, the group treated with the DGK-dKO iCAR-T_{CTL} genetically modified with mbIL-15 showed a remarkably longer survival time (Figs. 3B and C) and a remarkably higher cell survival performance (Figs. 3D and E) compared to the other groups.

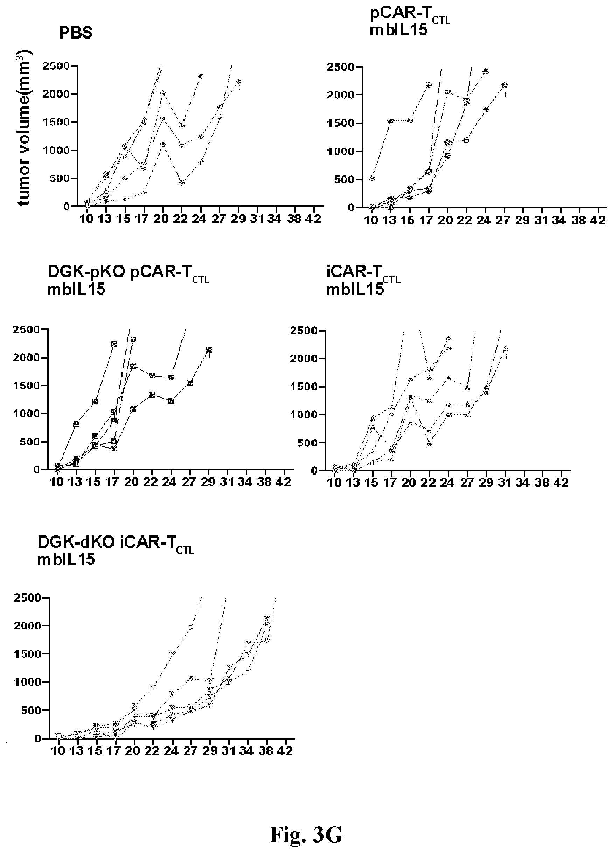

Further, in order to test the cells under clinically significant conditions, each type of CAR-T_{CTL} cells in an amount of 1×10⁶ cells were intravenously injected to a subcutaneous tumor model to which JHH7 had been preliminarily inoculated (Fig. 3F). As a result, similarly to the result obtained with the intraperitoneal injection model, the DGK-dKO iCAR-T_{CTL} genetically modified with mbIL-15 showed a remarkably higher tumor suppression performance and survival performance compared to the other groups (Figs. 3G to I). In an FCM analysis of peripheral blood that was carried out on Day 28 after the injection, the DGK-dKO iCAR-T_{CTL} genetically modified with mbIL-15 was the only cells detected among all groups (Fig. 3J)

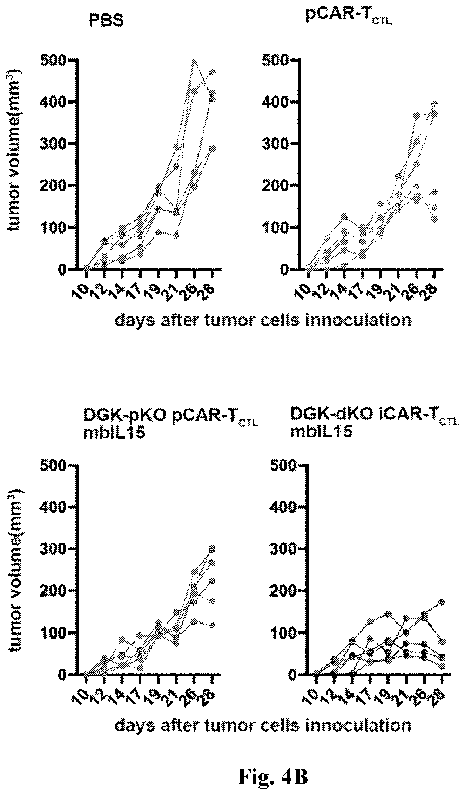

Further, in order to test the anti-tumor effect on an established and palpable tumor, each type of CAR-T_{CTL} cells in an amount of 1×10⁷ cells were intravenously injected to a subcutaneous tumor animal model 10 days after inoculation of SK-Hep-GPC3 (Fig. 4A). As a result, the DGK-dKO iCAR-T_{CTL} genetically modified with mbIL-15 showed a significantly higher tumor growth-suppressing effect compared to the other groups (Figs. 4B and C).

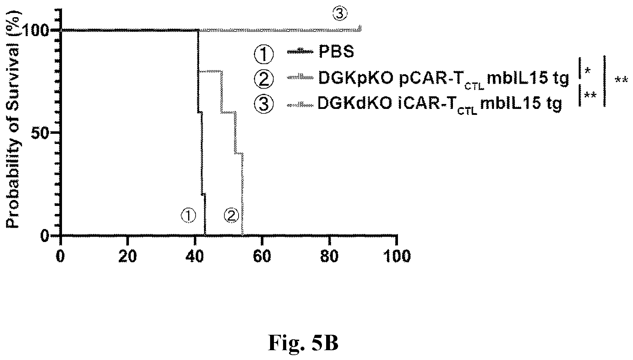

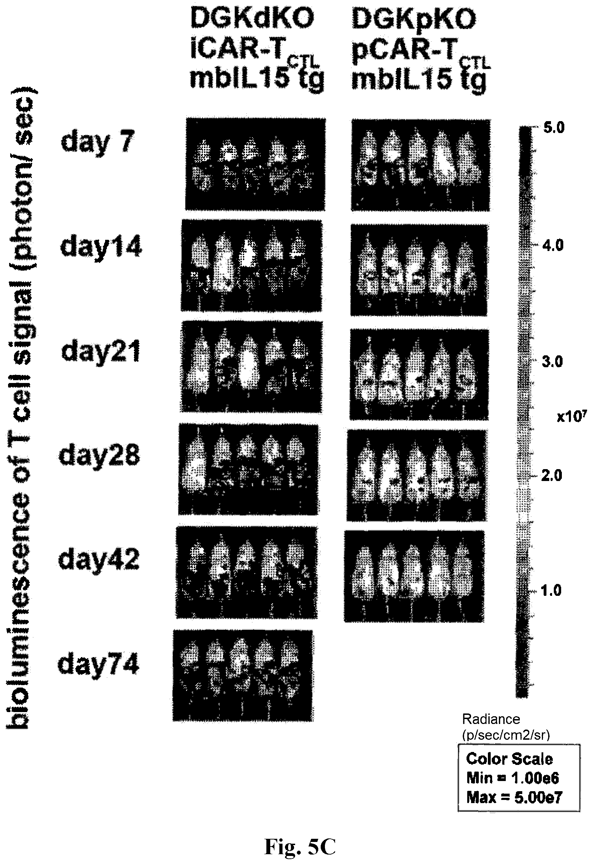

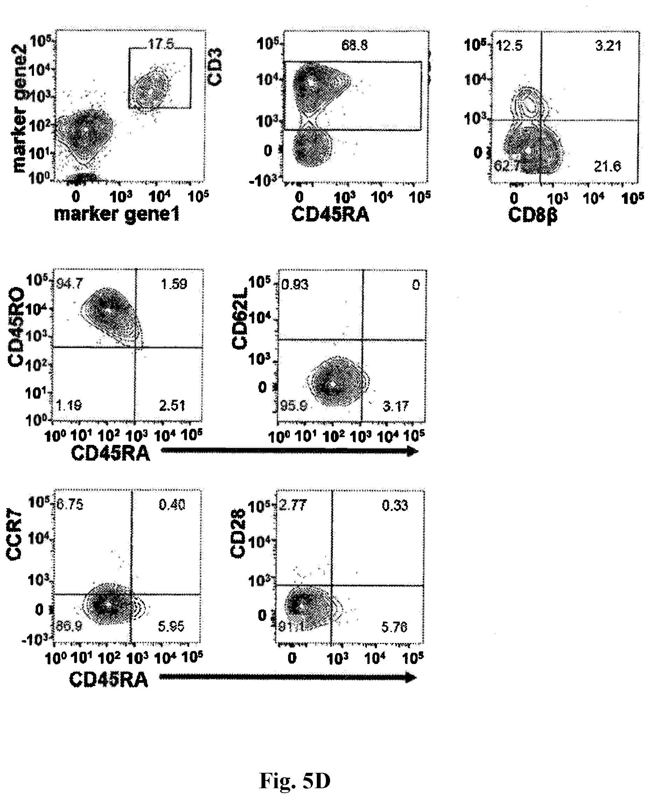

Further, each type of CAR-T_{CTL} cells in an amount of 1×10⁷ cells were intraperitoneally injected to a peritoneal dissemination animal model 10 days after inoculation of KOC7c (Fig. 5A). The group treated with the DGK-dKO iCAR-T_{CTL} genetically modified with mbIL-15 showed a remarkably longer survival time (Fig. 5B) and a remarkably higher cell survival performance (Fig. 5C) compared to the other groups. Further, according to an FCM analysis of ascites that was carried out on Day 80 after the injection, the effector memory phenotype was maintained in the group treated with the DGK-dKO iCAR-T_{CTL} genetically modified with mbIL-15 (Fig. 5D).

The above results strongly suggested that, by carrying out these modifications, iCAR-T_{CTL} can be used as a platform for allogeneic therapy in immunotherapy using regenerated T cells in which CAR is genetically modified.

## Claims

1. An immunocompetent cell having decreased diacylglycerol kinase activity, wherein said cell expresses a fusion protein (IL-15/IL-15Rα) comprising IL-15 and an IL-15 receptor α subunit (IL-15Rα), and expresses a chimeric antigen receptor.

2. The immunocompetent cell according to claim 1, wherein said cell comprises one or more kinds of diacylglycerol kinase genes having a mutation(s) that decrease(s) diacylglycerol kinase activity in a cell.

3. The immunocompetent cell according to claim 1 or 2, wherein the fusion protein is a transmembrane protein.

4. The immunocompetent cell according to any one of claims 1 to 3, wherein said cell is CD5-positive and CD8β-positive.

5. The immunocompetent cell according to any one of claims 1 to 4, wherein said cell is a T cell.

6. The immunocompetent cell according to any one of claims 1 to 5, wherein said cell is obtainable by differentiation induction from a pluripotent stem cell.

7. The immunocompetent cell according to claim 6, wherein the pluripotent stem cell is an induced pluripotent stem cell.

8. A pharmaceutical composition comprising the immunocompetent cell according to any one of claims 1 to 7.

9. The pharmaceutical composition according to claim 8, for treatment of cancer.

10. The pharmaceutical composition according to claim 9, wherein the cancer is solid cancer.

11. A method for treating cancer, comprising the step of administering a therapeutically effective amount of the immunocompetent cell according to any one of claims 1 to 7 to a subject in need of treatment.

12. Use of the immunocompetent cell according to any one of claims 1 to 7 in the manufacture of a pharmaceutical composition for cancer treatment.

13. The immunocompetent cell according to any one of claims 1 to 7 for use in treatment of cancer.

14. A method of producing an immunocompetent cell that expresses a chimeric antigen receptor, comprising the step of:
(a) providing an immunocompetent cell that expresses a chimeric antigen receptor;
wherein the immunocompetent cell has decreased diacylglycerol kinase activity, and expresses a fusion protein (IL-15/IL-15Rα) comprising IL-15 and an IL-15 receptor α subunit (IL-15Rα).

15. The method according to claim 14, comprising the step of:
(b) allowing a pluripotent stem cell to differentiate into an immunocompetent cell;
before Step (a).

16. The method according to claim 15, wherein the cell obtained in Step (b) expresses a chimeric antigen receptor.

17. The method according to claim 16, wherein a pluripotent stem cell that expresses a chimeric antigen receptor is used in Step (b), or a chimeric antigen receptor is allowed to be expressed in any stage of Step (b).

18. The method according to any one of claims 15 to 17, wherein the cell obtained in Step (b) has decreased diacylglycerol kinase activity.

19. The method according to claim 18, wherein a pluripotent stem cell having decreased diacylglycerol kinase activity is used in Step (b), or a modification that decreases diacylglycerol kinase activity is carried out in any stage of Step (b).

20. The method according to any one of claims 15 to 19, wherein the cell obtained in Step (b) expresses a fusion protein (IL-15/IL-15Rα) comprising IL-15 and an IL-15 receptor α subunit (IL-15Rα).

21. The method according to claim 20, wherein a pluripotent stem cell that expresses IL-15/IL-15Rα is used in Step (b), or IL-15/IL-15Rα is allowed to be expressed in any stage of Step (b).
